# FASCICULE DE BREVET EUROPEEN

(11) **EP 1 645 247 B1**
(45) Date de publication et mention de la délivrance du brevet: **23.07.2008**
(21) Numéro de dépôt: 05370031.6
(22) Date de dépôt: 07.10.2005
(51) Int. Cl.: A61F 2/08, A61L 27/58

(54) **Dispositif d'ancrage d'un ligament sur une structure osseuse**
Befestigungsvorrichtung für ein Band auf einer Knochenstruktur
Device for anchoring a ligament on a bone structure

(30) Priorité: 08.10.2004 FR 0410666
(43) Date de publication de la demande: 12.04.2006
(73) Titulaire: Charles, Hugues, 59420 Mouvaux (FR)
(72) Inventeur: Charles, Hugues, 59420 Mouvaux (FR)
(74) Mandataire: Hennion, Jean-Claude

(56) Documents cités:
- EP-A- 1 033 115
- WO-A-02/32345
- DE-U1- 9 409 972
- US-A- 5 354 298

## Description

L'invention a trait à un dispositif d'ancrage d'un ligament sur une structure osseuse, notamment destiné à être utilisé dans des opérations de ligamentoplastie Le document EP-A-1033115 est l'état de la technique le plus proche et définit le préambule de la revendication 1.

L'invention trouvera principalement son application dans des opérations chirurgicales permettant la réparation de la coiffe des rotateurs ; toutefois, le dispositif d'ancrage pourra également être utilisé dans des opérations de réparation d'autres articulations et notamment l'articulation du genou.

Le dispositif d'ancrage sera utilisé pour réaliser la liaison entre l'extrémité d'un ligament et l'os sur lequel il était fixé initialement.

En effet, lorsque la liaison entre le ligament et l'os est rompue, le ligament ne peut se raccrocher sans intervention sur l'os et nécessite l'emploi de dispositifs d'ancrages qui viennent faire la liaison entre l'os et le ligament soit directement, soit indirectement par l'intermédiaire de fils de suture.

Pour remplir ces fonctions, le dispositif d'ancrage doit présenter un certain nombre de caractéristiques de manière à :
- réaliser un bon ancrage sur l'os,
- présenter un profil bas au niveau de la surface de l'os pour éviter des détériorations de tissus ou la rupture au niveau de la tête d'agrafe,
- avoir une bonne fixation soit du ligament, soit des fils de suture tout en permettant un coulissement de ces derniers,
- présenter une résistance importante à la traction exercée par le ligament,
- éviter le cisaillement osseux,
- autoriser un contact important le tendon et l'os de manière à permettre une surface de cicatrisation importante,
- permettre l'utilisation de matériaux résorbables,
- permettre la réalisation des noeuds de suture sur ledit dispositif d'ancrage.

Différents dispositifs d'ancrage sont connus et commercialisés mais ne permettent pas de réaliser de manière satisfaisante et combinée les caractéristiques nécessaires précitées.

On connaît notamment du document WO-00/64378, un dispositif d'ancrage d'un ligament sur une structure osseuse composée de deux branches se croisant pour former un anneau et comportant des pattes portées par l'extrémité des branches permettant l'accrochage sur l'os.

Les essais sur la base de ce type de ce dispositif ont démontré de bons résultats ; cependant, il présente différents inconvénients parmi lesquels l'impossibilité compte tenu de sa structure de réaliser ce dispositif dans des matériaux résorbables ou encore l'impossibilité de réaliser des noeuds de suture sur ledit dispositif.

La présente invention a pour but de pallier aux inconvénients des dispositifs d'ancrage connus et de proposer à cet effet un dispositif d'ancrage d'un ligament sur une structure osseuse présentant une surface d'appui importante au niveau de la structure osseuse et dont la structure facilite le positionnement et le coulissement direct du ligament ou du fil de suture.

Un autre but de la présente invention est de proposer un dispositif d'ancrage dans lequel il est possible de réaliser au niveau du dispositif d'ancrage des noeuds de suture.

Un autre objet de la présente invention est de proposer un dispositif d'ancrage d'un ligament réalisable en un ou plusieurs matériaux résorbables.

L'invention a ainsi pour objet un dispositif d'ancrage d'un ligament sur une structure osseuse comme défini par la revendication 1 comprenant un corps allongé et une collerette, ladite collerette étant solidaire du corps allongé.

Selon l'invention, le corps allongé comporte une fente longitudinale avec une partie supérieure débouchant sur la surface supérieure dudit corps allongé permettant l'introduction du fils de suture ou du ligament, et une partie inférieure permettant le coulissement dudit fil de suture ou du ligament.

D'autres caractéristiques et avantages de l'invention apparaîtront plus clairement à la lecture de la description ci-après d'un exemple préféré de réalisation, dans lequel la description n'est donnée qu'à titre d'exemple non limitatif et en référence aux dessins annexés parmi lesquels :
- la figure 1 représente, selon une vue en perspective, un exemple de réalisation du dispositif d'ancrage conforme à l'invention,
- la figure 2 est une vue de dessus de l'exemple de la figure 1,
- les figures 3 et 4 représentent des vues de côté du dispositif d'ancrage des figures 1 et 2.

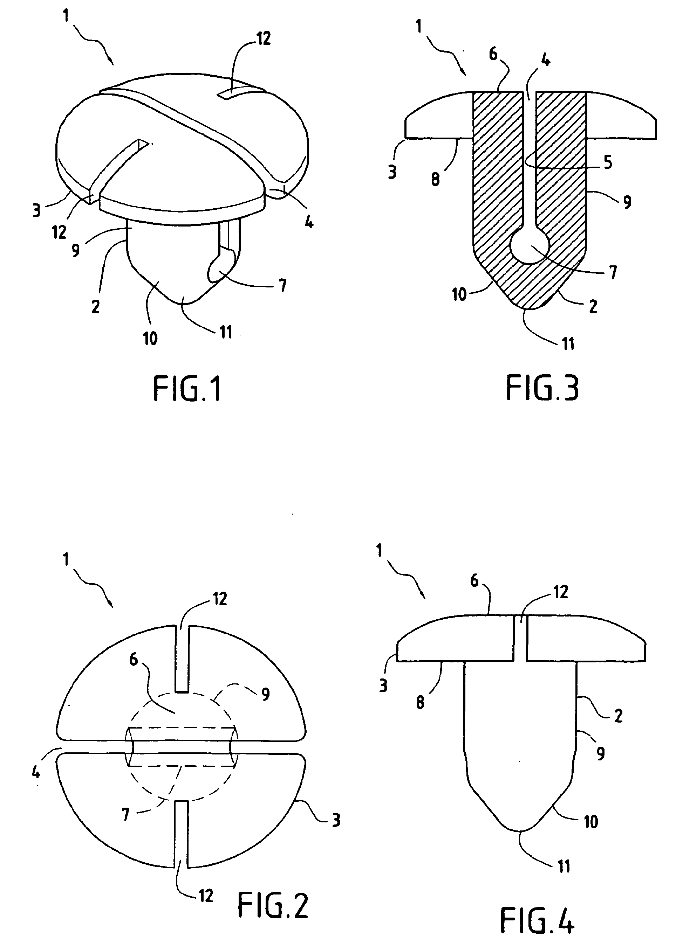

En se reportant principalement à la figure 3, on voit que le dispositif d'ancrage 1 comprend un corps allongé 2 et une collerette 3.

Il est important de signaler à ce niveau que la collerette 3 et le corps allongé 2 peuvent être formés d'une seule pièce.

Toutefois, en fonction de la production des pièces ou de leur utilisation, on pourra choisir de réaliser le corps et la collerette 3 séparément dans un matériau identique ou non.

Le corps allongé 2 comporte une fente longitudinale 4 avec une partie supérieure 5 débouchant sur la surface supérieure 6 dudit corps allongé 2.

Les dimensions de cette partie supérieure 5 permettent l'introduction directe du ligament ou du fil de suture.

Ladite fente 4 a également une partie inférieure 7 dont les dimensions permettent le coulissement direct du ligament ou du fil de suture.

On voit en se reportant également aux figures 2 et 3 que la collerette 3 présente une surface d'appui 8 importante, cette surface d'appui 8 venant en contact avec la corticale, c'est-à-dire la partie la plus résistante de l'os et permettant ainsi de conférer au dispositif d'ancrage 1 une résistance à la traction élevée.

Le corps allongé 2 présente une première partie cylindrique 9 prolongée par une seconde partie conique 10.

La partie conique permet une introduction aisée du dispositif d'ancrage 1 dans le puit formé dans l'os, la section circulaire de la partie cylindrique 9 permettant un coulissement aisé du dispositif d'ancrage 1 à l'intérieur du puit.

Il est également important de souligner qu'avantageusement, l'extrémité 11 de la partie conique 10 est arrondie de manière notamment à éviter toute usure des fils de suture ou des ligaments en cas de contact avec cette extrémité 11.

Dans l'exemple des figures 1 à 4, la collerette 3 est positionnée à un angle de 90° par rapport au corps allongé 2.

Toutefois, en fonction de l'angle formé entre le puit et la surface de l'os, on pourra imaginer d'utiliser des dispositifs d'ancrage 1 dont la collerette 3 forme un angle compris entre 45° et 90° par rapport audit corps allongé 2.

De manière avantageuse, on prévoira également que la hauteur de la collerette 3 soit peu importante au regard des dimensions de sa surface d'appui 8 permettant à la collerette 3 de présenter un profil bas.

Cette disposition permet de limiter les risques de détérioration des tissus avoisinants le lieu d'ancrage et de limiter également les risques d'arrachement dudit dispositif d'ancrage 1.

A cet effet, on pourra également noter qu'avantageusement, ladite collerette 3 présentera des angles adoucis.

Selon une caractéristique avantageuse de l'invention, le corps allongé 2 et la collerette 3 sont réalisés dans un matériau résorbable.

A ce sujet, on pourra notamment réaliser ledit corps de la collerette 3 avec un polymère de l'acide L-lactique.

Toutefois, il est évident que d'autres matériaux résorbables pouvant être utilisés dans le domaine médical pourront également être envisagés par l'homme du métier.

Le fonctionnement du dispositif d'ancrage 1 est le suivant :
- dans un premier temps, le chirurgien utilise un ancillaire pour traverser la structure osseuse sur lequel le fil de suture a été placé,
- le chirurgien procède ensuite au raccordement entre le ligament et le fil de suture puis le fil est repris et décroché de l'ancillaire pour être placé sur le dispositif d'ancrage 1.

La prise en main du dispositif d'ancrage 1 est facilitée par les dimensions de la collerette 3 rendant aisée l'introduction dans la fente 4 du fil de suture au bout du ligament.

Le fil ou le ligament va ensuite être poussé sur la longueur de la partie supérieure 5 de la fente 4 pour être déposé dans sa partie inférieure 7.

Il est à noter que pour faciliter l'introduction du fil ou ligament dans la fente 4, les bords de la surface supérieure 6 au voisinage de la fente 4 seront arrondis.

Selon un exemple de réalisation des figures 1 à 4, la partie inférieure 7 de la fente 4 présente une épaisseur maximale supérieure à celle de la partie supérieure 5.

En effet, cette partie inférieure 7 doit permettre le coulissement du fil ou du ligament de manière à rendre possible son réglage en longueur et en tension.

La partie supérieure 5 de la fente a une épaisseur constante ; ceci permet de descendre le fil ou le ligament ou éventuellement de le retirer sans qu'aucune aspérité se présente dans la montée ou la descente de la fente 4.

L'épaisseur de la fente 4 dans cette zone 5 sera avantageusement comprise entre 0,4 et 0,6 mm.

La partie inférieure 7 sera de préférence cylindrique, l'axe du cylindre étant sensiblement perpendiculaire au plan de la fente 4.

On utilisera un diamètre de cylindre supérieur au diamètre du fil de suture ou du ligament permettant de faire glisser ce dernier latéralement.

De manière avantageuse, le diamètre du cylindre sera compris entre 1,3 et 1,7 mm.

Lorsque le chirurgien souhaite avec le dispositif d'ancrage classique nouer les sutures, il le fait au dessus du dispositif d'ancrage 1 lorsque la structure de ce dernier le permet.

Dans la présente invention, on prévoit telles que présentées dans les figures 1 à 4 des rainures 12.

Ces rainures 12 permettent au chirurgien de réaliser le nouage du fil de suture en dessous de la collerette 3.

De manière avantageuse, ces rainures 12 sont disposées perpendiculairement au plan de la fente 4.

Les rainures 12 pourront toutefois également être disposées en oblique notamment lorsque l'ancrage est fixé par le fil de suture faisant le tour de l'os, cette disposition en oblique permettant d'éviter un glissement du fil hors des rainures.

Lorsque le dispositif d'ancrage 1 est placé dans le puit avec le placement des ligaments ou fils de suture effectués, on comblera le puit par un greffon osseux, le dispositif d'ancrage 1 étant alors complètement solidaire de l'ensemble greffon osseux/os.

Bien entendu, d'autres modes de réalisation à la portée de l'homme de l'art auraient pu être envisagés sans pour autant sortir du cadre de l'invention définie par les revendications ci-après.

## Revendications

1. Dispositif d'ancrage d'un ligament sur une structure osseuse comprenant un corps allongé (2) et une collerette (3), solidaire dudit corps allongé (2), le corps allongé (2) comportant une fente longitudinale (4) avec une partie supérieure (5) débouchant sur la surface supérieure (6) dudit corps allongé (2) permettant l'introduction du fil de suture ou du ligament et une partie inférieure (7) permettant le coulissement dudit fil de suture ou du ligament, **caractérisé en ce que** la collerette (3) présente une surface d'appui (8) apte à venir en contact avec la corticale.

2. Dispositif d'ancrage selon la revendication 1, dans lequel la partie inférieure (7) de la fente (4) présente une épaisseur maximale supérieure à celle de la partie supérieure (5) de la fente (4).

3. Dispositif d'ancrage selon la revendication 1 ou 2, dans lequel la partie inférieure (7) est cylindrique, l'axe du cylindre étant sensiblement perpendiculaire au plan de la fente (4).

4. Dispositif d'ancrage selon la revendication 3, dans lequel le cylindre a un diamètre compris entre 1,3 et 1,7 mm.

5. Dispositif d'ancrage selon l'une quelconque des revendications 1 à 4, dans lequel la partie supérieure (5) de la fente (4) a une épaisseur constante.

6. Dispositif d'ancrage selon la revendication 5, dans lequel l'épaisseur de la partie supérieure (5) de la fente est comprise entre 0,4 et 0,6 mm.

7. Dispositif d'ancrage selon l'une quelconque des revendications 1 à 6, dans lequel ledit corps allongé (2) présente une première partie cylindrique (9) prolongée par une seconde partie conique (10).

8. Dispositif d'ancrage selon la revendication 7, dans lequel l'extrémité (11) de la partie conique du corps allongé (2) est arrondie.

9. Dispositif d'ancrage selon l'une quelconque des revendications 1 à 8, dans lequel la collerette (3) comprend au moins deux rainures (12) permettant la fixation des fils de suture en dessous de ladite collerette (3).

10. Dispositif d'ancrage selon l'une quelconque des revendications 1 à 9, dans lequel l'axe de chacune desdites rainures (12) est perpendiculaire au plan de la fente (4).

11. Dispositif d'ancrage selon l'une quelconque des revendications 1 à 10, dans lequel la collerette (3) est positionnée à un angle compris entre 45 et 90° par rapport au corps allongé (2).

12. Dispositif d'ancrage selon l'une quelconque des revendications 1 à 11, dans lequel le corps allongé (2) et la collerette (3) sont réalisés dans un matériau résorbable.

13. Dispositif d'ancrage selon la revendication 12, dans lequel ledit matériau résorbable est un polymère de l'acide L-lactique.

14. Dispositif d'ancrage selon l'une quelconque des revendications 1 à 12, dans lequel la collerette (3) et le corps allongé (2) sont formés d'une seule pièce.

## Claims

1. A device for anchoring a ligament to a bony structure, which includes an elongated body (2) and a small flange (3) attached to the said elongated body (2), wherein the elongated body (2) includes a longitudinal slot (4) with an upper part (5) that opens onto the top surface (6) of the said elongated body (2) allowing insertion of the suture filament or of the ligament, and a lower part (7) allowing the sliding of the said suture filament or of the ligament, **characterised in that** the small flange (3) has a bearing surface (8) adapted to come into contact with the cortex.

2. The anchoring device according to claim 1, wherein the lower part (7) of the slot (4) has a maximum thickness that is greater than that of the top part (5) of the slot (4).

3. The anchoring device according to claim 1 or 2, wherein the lower part (7) is cylindrical, the axis of the cylinder being substantially perpendicular to the plane of the slot (4).

4. The anchoring device according to claim 3, wherein the cylinder has a diameter of between 1.3 and 1.7 mm.

5. The anchoring device according any of claims 1 to 4, wherein the top part (5) of the slot (4) is of constant thickness.

6. The anchoring device according to claim 5, wherein the thickness of the top part (5) of the slot is between 0.4 and 0.6 mm.

7. The anchoring device according to any of claims 1 to 6, wherein the said elongated body (2) has a first cylindrical part (9) which is extended by a second conical part (10).

8. The anchoring device according to claim 7, wherein the end (11) of the conical part of the elongated body (2) is rounded.

9. The anchoring device according to any of claims 1 to 8, wherein the small flange (3) includes at least two grooves (12) that allow the attachment of the suture filament below the said small flange (3).

10. The anchoring device according to any of claims 1 to 9, wherein the axis of each of the said grooves (12) is perpendicular to the plane of the slot (4).

11. The anchoring device according to any of claims 1 to 10, wherein the small flange (3) is positioned at an angle of between 45 and 90 degrees to the elongated body (2).

12. The anchoring device according to any of claims 1 to 11, wherein the elongated body (2) and the small flange (3) are made from a resorptive material.

13. The anchoring device according to claim 12, wherein the said resorptive material is an L-lactic acid polymer.

14. The anchoring device according to any of claims 1 to 12, wherein the small flange (3) and the elongated body (2) are formed in one piece.

## Patentansprüche

1. Vorrichtung zur Verankerung eines Bandes an einer Knochenstruktur, mit einem länglichen Körper (2) und einem Kragen (3), welcher mit dem länglichen Körper (2) fest verbunden ist, wobei der längliche Körper (2) einen Längsschlitz (4) mit einem an der Oberseite (6) des länglichen Körpers (2) ausmündenden oberen Teil (5), der das Einführen des Nahtfadens oder des Bandes ermöglicht, sowie mit einem unteren Teil (7), der das Gleiten des Nahtfadens oder des Bandes ermöglicht, umfaßt, **dadurch gekennzeichnet, daß** der Kragen (3) eine Auflagefläche (8) aufweist, die geeignet ist, mit der Kortikalis in Kontakt zu kommen.

2. Verankerungsvorrichtung nach Anspruch 1, bei welcher der untere Teil (7) des Schlitzes (4) eine maximale Dicke aufweist, die größer als die des oberen Teils (5) des Schlitzes (4) ist.

3. Verankerungsvorrichtung nach Anspruch 1 oder 2, bei welcher der untere Teil (7) zylindrisch ist, wobei die Achse des Zylinders im wesentlichen senkrecht zur Ebene des Schlitzes (4) verläuft.

4. Verankerungsvorrichtung nach Anspruch 3, bei welcher der Zylinder einen Durchmesser von 1,3 bis 1,7 mm aufweist.

5. Verankerungsvorrichtung nach irgendeinem der Ansprüche 1 bis 4, bei welcher der obere Teil (5) des Schlitzes (4) eine konstante Dicke aufweist.

6. Verankerungsvorrichtung nach Anspruch 5, bei welcher die Dicke des oberen Teils (5) des Schlitzes zwischen 0,4 und 0,6 mm beträgt.

7. Verankerungsvorrichtung nach irgendeinem der Ansprüche 1 bis 6, bei welcher der längliche Körper (2) einen ersten zylindrischen Teil (9) aufweist, der durch einen zweiten kegelförmigen Teil (10) verlängert ist.

8. Verankerungsvorrichtung nach Anspruch 7, bei welcher das Ende (11) des kegelförmigen Teils des länglichen Körpers (2) abgerundet ist.

9. Verankerungsvorrichtung nach irgendeinem der Ansprüche 1 bis 8, bei welcher der Kragen (3) wenigstens zwei Nuten (12) aufweist, die das Fixieren der Nahtfäden unterhalb des Kragens (3) ermöglichen.

10. Verankerungsvorrichtung nach irgendeinem der Ansprüche 1 bis 9, bei welcher die Achse einer jeden der Nuten (12) senkrecht zur Ebene des Schlitzes (4) verläuft.

11. Verankerungsvorrichtung nach irgendeinem der Ansprüche 1 bis 10, bei welcher der Kragen (3) in einem Winkel zwischen 45 und 90° gegenüber dem länglichen Körper (2) angeordnet ist.

12. Verankerungsvorrichtung nach irgendeinem der Ansprüche 1 bis 11, bei welcher der längliche Körper (2) und der Kragen (3) aus einem resorbierbaren Material gefertigt sind.

13. Verankerungsvorrichtung nach Anspruch 12, bei welcher das genannte resorbierbare Material ein Polymer der L-Milchsäure ist.

14. Verankerungsvorrichtung nach irgendeinem der Ansprüche 1 bis 12, bei welcher der Kragen (3) und der längliche Körper (2) einstückig ausgebildet sind.
